# EUROPÄISCHE PATENTSCHRIFT

(11) **EP 1 034 160 B1**
(45) Veröffentlichungstag und Bekanntmachung des Hinweises auf die Patenterteilung: **05.06.2002**
(21) Anmeldenummer: 98956710.2
(22) Anmeldetag: 23.11.1998
(51) Int. Cl.: C07C 67/03, C07C 69/52, B01J 19/00

(54) **VERFAHREN ZUR HERSTELLUNG VON FETTSÄUREMETHYLESTER UND ANLAGE ZUR DURCHFÜHRUNG DES VERFAHRENS**
METHOD FOR PRODUCING FATTY ACID METHYL ESTER AND EQUIPMENT FOR REALISING THE SAME
PROCEDE DE PRODUCTION D'ESTER METHYLIQUE D'ACIDE GRAS ET INSTALLATION POUR METTRE EN OEUVRE LEDIT PROCEDE

(30) Priorität: 24.11.1997 AT 199097; 30.10.1998 AT 180798
(43) Veröffentlichungstag der Anmeldung: 13.09.2000
(73) Patentinhaber: Energea Umwelttechnologie GmbH, 1010 Wien (AT)
(72) Erfinder: ERGÜN, Nurhan, A-1010 Wien (AT); PANNING, Peter, A-7033 Pöttsching (AT)
(74) Vertreter: Krause, Peter
(86) Internationale Anmeldenummer: AT9800284
(87) Internationale Veröffentlichungsnummer: WO9926913

(56) Entgegenhaltungen:
- EP-A- 0 041 204
- EP-A- 0 523 767
- WO-A-92/00268

## Beschreibung

Die Erfindung betrifft ein Verfahren zur Herstellung von Fettsäuremethylester, insbesondere Dieselkraftstoff für Fahrzeuge, wobei höhere, gesättigte und ungesättigte Fette pflanzlichen und/oder tierischen Ursprungs mit einer Lösung von starken Laugen, insbesondere Kalilauge, in Alkohol, insbesondere in Methanol, versetzt werden und die beiden Komponenten in einer Reaktionsstrecke durch Emulgierung der Mischung über eine Grenzflächenreaktion, wobei die Fette in Fettsäuremethylester umgeestert werden, ihren chemischen Gleichgewichtszustand erreichen und nach Erreichen des Gleichgewichtszustand die Rückstände, wie Triglyceride, Glycerin, Seife, nicht umgeesterte Fette u. dgl., vom Fettsäuremethylester getrennt werden sowie eine Anlage zur Durchführung des Verfahrens.

Derartige Verfahren sind bekannt, wobei die in Alkohol gelöste Lauge mit dem Fett bzw. Öl in einem Rührtank etwa 20 Minuten bis zu einer Stunde gerührt wird. Nach dem Rührvorgang läßt man die Mischung ruhen. Dieser Absetzvorgang dauert etwa 5-8 Stunden. Nach dem Absetzen wird die Glycerinphase abgezogen. Nun wird die Flüssigphase gegebenenfalls wieder mit der Methanol-Kalilauge-Lösung versetzt und der Vorgang des Rührens und Abziehens entsprechend wiederholt. Die umgeesterte Flüssigkeit wird anschließend mit Phosphorsäure, Zitronensäure oder andere Säuren neutralisiert, wobei sich dabei Seife und Kaliumsalze der Säuren absetzen. In manchen Fällen wird es auch mit Wasser gewaschen, wobei das Wasser die Seife, Kalilauge u. dgl. aufnimmt. Diese Phase wird ebenfalls abgezogen. Anschließend können alle erdenklichen Reinigungsschritte durchgeführt werden. Auch der Verfahrensschritt Strippen ist möglich, wobei in einem Waschturm Luft im Gegenstrom zum Ester geführt wird. Als Nachteil dieses Verfahrens, dem ein Niederdruckumesterungsprozeß zugrunde liegt, ist die lange Herstellzeit zu werten. Abgesehen vom Rührvorgang sind in den Absetzphasen lange Stillstandszeiten erforderlich. Darüber hinaus ist auch ein großer Platzbedarf für die Behälter notwendig. Ebenfalls nachteilig hat sich der große statische Aufwand für die schweren Konstruktionen und Fundamente ausgewirkt.

Ein weiterer Nachteil des bekannten Verfahrens ist darin zu sehen, daß immer eine mehr oder minder große Verschmutzung des Kraftstoffes gegeben ist.

Aus der Literaturstelle in **Falbe und Regitz, RÖMPP Chemie** Lexikon, 9. erw. Aufl., Bd.2, Georg Thieme Verlag Stuttgart-New York 1990, Seite 1343 ist ein Verfahren zur Herstellung von Fettsäuremethylester bekannt, bei dem nach Absetzung der Glycerin 10 Lösung in einem Seperator eine Destillation zur Reinigung und gegebenenfalls eine Fraktionierung des Methylesters durchgeführt wird. Ferner kann eine Erhöhung der Reaktionsgeschwindigkeit bei der Umesterung durch Temperaturerhöhung sowie durch alkalische oder saure Katalysatoren erreicht werden. Nachteilig bei diesem Verfahren ist, daß durch die Absetzphase im Seperator für die Glycerin-Lösung und auch die Möglichkeit der erhöhten Reaktionsgeschwindigkeit keine nennenswerte Verkürzung der Herstelldauer, wie sie aus dem obigen Stand der Technik bekannt ist, eintritt.

Ferner ist aus der AT-PS 398 777 ein Verfahren zur Reinigung von rohen Pflanzenölestern bekannt, wobei der Pflanzenölester durch alkalische Umesterung erhalten wird. Die Umesterung erfolgt mit Methanol im Überschuß, unter Zugabe von Kaliumhydroxid als Katalysator. Der rohe Pflanzenölester wird mit Wasserdampf behandelt, wobei eine Glycerinphase gebildet wird, die abgezogen wird. Bei diesem Verfahren wird zur Umesterung intensiv gerührt und der ausgetriebene Alkohol kann nach der Rückgewinnung durch eine Destillationskolonne wiederverwertet werden.

Es sind aber auch Verfahren bekannt, denen ein Hochdruckumesterungsprozeß zugrunde liegt. Dabei erfolgt die Umesterung in einem Autoklaven mit einer relativ kurzen Reaktionszeit. Der Nachteil derartiger Verfahren bzw. Anlagen liegt darin, daß eine wirtschaftliche Herstellung des Fettsäuremethylesters, also beispielsweise eines Dieselkraftstoffes für Fahrzeuge, absolut nicht möglich ist.

Darüber hinaus ist es auch bekannt, den Umesterungsprozeß in zwei Stufen durchzuführen. Dabei ist die quantitative und qualitative Ausbeute sicherlich höher als bei einer Umesterung in einem Arbeitsvorgang, jedoch ist auch hier eine Wirtschaftlichkeit nicht zu erzielen, da hohe Anlage- und Herstellkosten gegeben sind.

Aufgabe der Erfindung ist es, ein Verfahren und eine Anlage zur Herstellung von Fettsäuremethylester, insbesondere von Dieselkraftstoff für Fahrzeuge, zu schaffen, das einerseits die Nachteile der bekannten Verfahren vermeidet und das anderseits eine rationelle Herstellung in einer wirtschaftlich vertretbaren Anlage, vorzugsweise in einer industriellen Großanlage, erlaubt, aber auch Kleinanlagen in die Wirtschaftlichkeit bringt.

Das erfindungsgemäße Verfahren ist dadurch gekennzeichnet, daß in der Reaktionsstrecke die Phasengrenzflächen der Mischung durch hohe bzw. starke, dynamische Turbulenzen vergrößert werden und daß die Umesterung unter hohem Druck durchgeführt wird, wobei gegebenenfalls die Umesterung unter hohem Druck beginnt und der Druck während der Umesterung abgebaut wird.

Mit der Erfindung ist es erstmals möglich, Dieselkraftstoff, sogenannten ÖKO-Diesel oder Biodiesel, mit allen seinen Vorteilen unter optimalen ökologischen Produktionsbedingungen herzustellen. Durch die Erfindung werden positive wirtschaftspolitische und ökologische Argumente geschaffen, die anregen, die Rolle von erneuerbarer Energie und Rohstoffen wieder intensiver zu überdenken.

Ein weiterer überraschender Vorteil ergibt sich durch die Erfindung, nämlich auch am Sektor der Abfallbeseitigung bzw. bei der Problemstoffentsorgung. Durch die Erfindung ist es auch möglich Altspeiseöl ökologisch wieder und weiter zu verwerten. Der Einsatz von Altspeiseöl ist beim erfindungsgemäßen Verfahren durch die hohe Reinheit der Endprodukte bedenkenlos möglich.

Mit der vorliegenden Erfindung wird die Möglichkeit geschaffen, die Reaktion durch die Vergrößerung der Grenzflächen und durch dynamische Vorgänge bei der Umesterung zu beschleunigen. Durch die hohen bzw. starken dynamischen Turbulenzen werden praktisch die Tropfen der Flüssigkeitsphasen wirksam verkleinert, wodurch also viel kleinere Tropfen entstehen, somit eine viel größere Oberfläche gegeben ist und das chemische Gleichgewicht schneller erreicht wird. Die Einstellung des Gleichgewichtes beträgt mitunter weniger wie eine Minute. Daraus resultiert eine immense Verkürzung der Herstellzeit. Dieses erfindungsgemäße Verfahren ist jedoch nicht für das sogenannte Absetzverfahren geeignet, da die Absetzzeiten durch die feine Verteilung der Tropfen zu lange dauern würden.

Durch den hohen Druck entsteht in den Verengungen der Reaktionsstrecke eine hohe Strömung, die wiederum am Ende der Verengung zu hohen Turbulenzen führt. Es ist erwiesen, daß die Länge der Reaktionsstrecke um so kürzer gewählt werden kann, je größer der Druckverlust ist. Es ist weiters erwiesen, daß der Druckverlust zugunsten der Vergrößerung der Phasengrenzflächen geht. Vorteilhaft ist dabei, daß der Druck am Beginn der Umesterung bis zu 200 bar betragen kann. Natürlich wird dadurch die Dynamik der Turbulenzen erhöht. Der Druckverlust wird im Laufe der Reaktionsstrecke in Grenzflächenvergrößerung und -dynamik verwertet.

Nach einem besonderen Merkmal der Erfindung werden die hohen bzw. starken dynamischen Turbulenzen durch physikalische Kräfte, beispielsweise mechanische Scherkräfte, wie einen Spaltemulsionsvorgang oder durch dynamische Emulgierung, beispielsweise über einen Turbulator, oder durch Kavitationsemulgation oder durch Ultraschall, erzeugt. Vorteilhaft ist dabei, daß durch die Scherkräfte, die durchaus mechanisch erzeugt werden können, viel stärkere Turbulenzen gegeben sind, die die Anzahl der Tropfen auf Kosten der Tropfengröße wesentlich erhöhen. Durch die Spalte beim Spaltemulsionsvorgang entsteht eine hohe Strömung der durchtretenden Flüssigkeit, die am Spaltende in hohe Turbulenzen bzw. Verwirbelungen übergeht. Diese Turbulenzen bzw. Verwirbelungen führen zu einer Vergrößerung der Phasengrenzflächen. Auch im Fall eines Turbulator führen die Turbulenzen bzw. Verwirbelungen zu einer Vergrößerung der Phasengrenzflächen der Mischung. Mit dem Verfahrensschritt der Kavitationsemulgation ist eine weitere Verkürzung der Reaktionszeit zu erzielen, da diese durch diese Optimierung nur mehr etwa 20 Sekunden beträgt. Mit einem Ultraschallgerät können in der Reaktionsstrecke ebenfalls große Grenzflächen erzeugt werden, durch eine weitere Teilung der Tropfen.

Gemäß einem besonderen Merkmal der Erfindung wird die Trennung der Phasen des Emulgates auf physikalischem Weg und nach der Trennung der Phasen des Emulgates eine Reinigung des Fettsäuremethylesters durchgeführt, wobei die Trennung der Phasen des Emulgates unter Ausnützung der Oberflächenkräfte, insbesondere über eine, gegebenenfalls mehrstufige, Filtration erfolgt und zur Filtrierung, gegebenenfalls lipophile und/oder hydrophile und/oder amphotere, Filter aus der Membrantechnologie, beispielsweise aus dem Ultra- und/oder Nano- und/oder Mikrobereich, eingesetzt werden. Mit diesem vorteilhaften Verfahrensschritt ist eine Abtrennung der einzelnen Phasen der Mischung zu erreichen, wobei durch die mechanischen Einrichtungen, wie beispielsweise Filtrationsanlagen, die aus dem Stand der Technik bekannte Absetzphase nicht mehr durchgeführt wird und durch rationelle, moderne Industriemethoden ersetzt wird. Prinzipiell kann durch den Verfahrensschritt, nämlich nach der Trennung der Phasen des Emulgates eine Reinigung des Fettsäuremethylesters durchzuführen, reiner Fettsäuremethylester erzielt werden. Die Trennung der Phasen des Emulgates wird unter Ausnützung der Oberflächenkräfte, insbesondere über eine Filtration durchgeführt. Durch diese Art der Trennung der Phasen der Mischung wird eine gute Wirtschaftlichkeit bei einer industriellen Herstellung erzielt. Die Trennung der Phasen des Emulgates wird über eine mehrstufige Filtration durchgeführt. Dabei fällt in der ersten Stufe die Glycerin-Phase und in einer zweiten Stufe die Triglyceride als Retendat an. In einer dritten Stufe wird das Methanol vom Fettsäuremethylester getrennt. Eine derartige Filteranlage wird auch als Cross-flow Filter ausgeführt. Durch diesen vorteilhaften Verfahrensschritt können beliebig viele Stufen durchlaufen und die gewünschte Reinheit erzielt werden. So ist es durchaus denkbar, je nach Fettsäurekette, Sommer- oder Winterdiesel als ÖKO- oder Biodiesel herzustellen. Es werden zur Filtrierung Filter aus der Membrantechnologie, beispielsweise aus dem Ultra- und/oder Nano- und/oder Mikrobereich, eingesetzt. Derartige Filter gewährleisten bei der heutigen Technologie einen einwandfreien Betrieb, wobei auch die Reinigung dieser Filter überaus einfach ist. Ferner werden lipophile und/oder hydrophile und/oder amphotere Filter eingesetzt. In Hinblick auf deren Aufgabe wird die Eigenschaft des Filters ausgewählt.

Nach einem besonderen Merkmal der Erfindung wird die Trennung der Phasen des Emulgates auf chemischem Weg durchgeführt, wobei nach Einstellung des Gleichgewichtes, gegebenenfalls nach der Trennung der Phasen des Emulgates, eine mehrstufige Destillation durchgeführt wird, die mindestens eine Vakuumdestillation oder mindestens eine Verdampfung, insbesondere eine Fallfilmverdampfung oder eine Dünnschichtverdampfung oder eine fraktionierte Kondensation umfaßt.. Auch ein derartiger Weg ist zur Trennung der Phasen vorteilhaft, wobei die aus dem Stand der Technik bekannten Absetzzeiten auf den Minutenbereich verkürzt werden. Nach Erreichung des Gleichgewichtes kann direkt eine Destillation durchgeführt werden, wobei auch gleichzeitig Methanol entfernt wird. Es ist aber auch denkbar, die Destillation nach der Filtration durchzuführen. Die mehrstufige Destillation umfaßt mindestens eine Vakuumdestillation. Mit diesem vorteilhaften Verfahrensschritt ist eine gezielte Abtrennung der einzelnen Stoffe zu erreichen, wobei durch das Vakuum die Temperaturen, bei denen das Verfahren durchgeführt wird, heruntergesetzt werden können. Prinzipiell kann durch die Destillation reiner Fettsäuremethylester erzielt werden. Die mehrstufige Destillation kann auch mindestens eine Verdampfung, insbesondere eine Fallfilmverdampfung umfassen. Durch diese Art der Verdampfung wird eine effektivere Auftrennung der abzuscheidenden Stoffe erzielt. Die mehrstufige Destillation kann aber auch mindestens eine Verdampfung, insbesondere eine Dünnschichtverdampfung umfassen. Auch mit dieser Verdampfungsart ist eine gezielte 20 Auftrennung der Stoffe zu erreichen. Darüber hinaus kann die mehrstufige Destillation auch eine fraktionierte Kondensation umfassen. Durch diesen vorteilhaften Verfahrensschritt können beliebig viele Stufen durchlaufen und die gewünschte Reinheit erzielt werden. So ist es durchaus denkbar, je nach Fettsäurekette, Sommer- oder Winterdiesel als ÖKO- oder Biodiesel herzustellen.

Gemäß einem weiteren besonderen Merkmal der Erfindung werden die nicht umgeesterten Anteile separiert und vor der Reaktionsstrecke den Fetten wieder zugeführt. Dadurch wird die Ausbeute an ökologischen Kraftstoff aus den eingesetzten Basisstoffen erhöht.

Die Aufgabe der Erfindung wird aber eigenständig auch durch eine Anlage zur Durchführung des Verfahrens gelöst. Die erfindungsgemäße Anlage ist dadurch gekennzeichnet, daß mindestens ein Behälter für die Fette sowie je ein Vorratsbehälter für die starke Lauge und für den Alkohol bzw. mindestens ein Mischbehälter für deren Lösung vorgesehen sind und mindestens der Behälter und der Mischbehälter mit der Reaktionsstrecke verbunden sind und daß der Reaktionsstrecke eine Einrichtung zur Trennung der Phasen des Emulgates nachgeschaltet ist. Mit dieser erfindungsgemäßen Anlage ist es erstmals möglich, das erfindungsgemäße Verfahren effizient und mit einem verschwindend geringen Anteil an Umweltbelastung durchzuführen. Die erfindungsgemäße Anlage hat den Vorteil, daß sie platzsparend und dadurch kostengünstig gebaut werden kann. Es ist ein Bau als industrielle Großanlage, die wirtschaftlich betrieben werden kann, möglich.

Nach einem besonderen Merkmal der Erfindung besteht die Reaktionsstrecke aus einem Statikmixer, wobei der Statikmixer, vorzugsweise ein mit verschieden großen Kugeln gefülltes Rohr und/oder gegebenenfalls Einbauten wie Leitbleche, Propeller, Widerstandskörper, od. dgl. aufweist. Durch diese Ausgestaltung der Erfindung werden mit einer einfachen Einrichtung hohe bzw. starke dynamische Turbulenzen für die Umesterungsphase erzielt. Dieses Standardgerät hat sich vorteilhaft im Einsatz in der erfindungsgemäßen Anlage bewährt. Dieses Gerät ist einfach im Aufbau und dadurch auch wartungsarm im Betrieb. Die Turbulenzen entstehen in erster Linie durch die rasche Strömung der Mischung um die Kugeln.

Nach einer Weiterbildung der Erfindung ist in der Reaktionsstrecke ein dynamischer Emulgator, beispielsweise ein Kavitationsemulgator oder ein Spaltemulgator oder ein Turbulator oder eine Mischform eines Spaltemulgators und eines Turbulators vorgesehen ist, die beispielsweise aus zwei sich relativ zueinander bewegender Scheiben besteht und die Zuführung des Emulgates mittig einer Scheibe erfolgt. Da ja aus der Reaktionsstrecke eine Emulsion austreten soll, das heißt eine Flüssigkeit die zwei Phasen aufweist, die durch die Braun'sche Molekularbewegung in Schwebe gehalten werden, ist eine derartige Einrichtung bestens geeignet, daß dieses Ziel sehr rasch erreicht wird. Ein Spaltemulgator hat den Vorteil, daß die Spaltbreite und/oder die Spaltlänge entsprechend den Erfordernissen frei gewählt werden kann bzw. können. Dadurch kann auch auf die nach dem Spalt erfolgenden Turbulenzen bzw. Verwirbelungen Einfluß genommen werden. Auch mit einem Turbulator kann in vorteilshafterweise eine Vergrößerung der Phasengrenzfläche in kurzer Zeit erzielt werden. Eine Mischform eines Spaltemulgators und eines Turbulators, die beispielsweise aus zwei sich relativ zueinander bewegender Scheiben besteht und die Zuführung des Emulgates mittig einer Scheibe erfolgt, hat den Vorteil, daß eine äußerst kurze Reaktionszeit erreichbar ist. Mit einem Kavitationsemulgator ist in vorteilhafterweise die kürzeste Reaktionszeit zu erreichen, ohne daß qualitative oder quantitative Einbußen hingenommen werden müssen.

Nach einer weiteren Ausgestaltung der Erfindung ist in der Reaktionsstrecke ein Ultraschallgerät vorgesehen. Die Integrierung eines Ultraschallgerätes hat sich als vorteilhaft erwiesen, da damit durch große Grenzflächen die Umesterung gezielt beschleunigt werden kann.

Gemäß einem ganz besonderen Merkmal der Erfindung ist die Einrichtung zum Trennen der Phasen des Emulgates eine, gegebenenfalls mehrstufige, Filtrationseinrichtung, wobei als Filtrationseinrichtung ein Oberflächenfilter in Membrantechnologie vorgesehen ist und der Oberflächenfilter aus einem porösen Trägerkörper und einer auf diesen Trägerkörper aufgebrachten Schicht, die als Membran wirkt, besteht und der Trägerkörper als Rohr ausgebildet ist und beispielsweise aus Aluminiumoxid, porösem Glas oder Silikaten besteht und die als Membran wirkende Schicht lipophile und/oder hydrophile und /oder amphotere Eigenschaften aufweist, wobei die als Membran wirkende Schicht eine Keramikmembran ist, die beispielsweise aus Titandioxid, Zirkondioxid, o. dgl. besteht und Poren im Nano- und/oder Mikrobereich. insbesondere mit einer Größe von 5-200 nm, aufweist oder die Filtrationseinrichtung ein Molekularsiebfilter bzw. eine Molekularsiebmembran ist bzw. umfaßt. Mit dieser vorteilhaften Einrichtung ist eine Abtrennung der einzelnen Phasen der Mischung zu erreichen, wobei durch die mechanischen Einrichtungen die aus dem Stand der Technik bekannte Absetzphase nicht mehr durchgeführt werden muß. Sie wird ersetzt durch Arbeitsgänge, die mit rationellen, modernen und industriellen Einrichtungen, Anlagen bzw. Geräten durchgeführt werden. Wenn als Filtrationseinrichtung ein Oberflächenfilter in Membrantechnologie vorgesehen ist, werden vorteilshafterweise beste Ergebnisse erzielt und zwar sowohl in qualitativer wie auch in quantitativer Hinsicht. Besteht der Oberflächenfilter aus einem porösen Trägerkörper und einer auf diesen Trägerkörper aufgebrachten Schicht, die als Membran wirkt, so kann die Anlage mit höchster Effizienz und die Energie optimal ausnützend betrieben werden. Der Oberflächenfilter kann natürlich auch als Platte ausgeführt werden. Bei einer Ausbildung des Trägerkörpers als Rohr ist der Vorteil darin zu sehen, daß ein kontinuierlicher Verfahrensablauf auch bei unterschiedlichen Zuflußmengen gewährleistet ist. Derartige Materialien, wie beispielsweise Aluminiumoxid, poröses Glas oder Silikate sind relativ einfach zu bearbeiten und haben sich im Verfahren bestens bewährt. Die als Membran wirkende Schicht weist lipophile und/oder hydrophile und /oder amphotere Eigenschaften auf. Durch die Wahl dieser Eigenschaften kann bestimmt werden, welche Phase der Mischung durch den Filter durchtritt und welche Phase als Retendat verbleibt. Die als Membran wirkende Schicht kann auch eine Keramikmembran sein, die beispielsweise aus Titandioxid, Zirkondioxid, Silizium oder Siliziumverbindungen o. dgl. besteht. Auf dieser Schicht mit den zitierten Materialien bildet sich eine Deckschicht aus Fettsäuremethylester, die beispielsweise die Glycerin-Phase nicht durchtreten läßt. Auf dieser Schicht aus Titandioxid bzw. Zirkondioxid bildet sich keine Glycerinschicht als Deckschicht aus.

Ferner kann die als Membran wirkende Schicht Poren im Nano- und/oder Mikrobereich, insbesondere mit einer Größe von 1-200 nm, aufweisen. Derartige Porengrößen sind mit den heutigen Technologien herstellbar und haben ausgezeichnete Ergebnisse geliefert. Die Filtrationseinrichtung kann auch ein Molekularsiebfilter bzw. eine Molekularsiebmembran umfassen. Mit derartigen speziellen Filtern ist überraschenderweise auch eine Trennung der nicht umgeesterten Fette vom Fettsäuremethylester möglich. Die Filtrationseinrichtung kann auch mehrstufig ausgebildet sein. Entsprechend des gewünschten Reinigungsgrades des Fettsäuremethylesters können mehrere Filter in Serie oder parallel angeordnet werden.

Der Reaktionsstrecke kann auch, gegebenenfalls nach der Einrichtung zur Trennung der Phasen, eine Destilliereinrichtung bestehend aus mindestens einem Verdampfer und einem Kondensator nachgeschaltet sein. Auch mit dieser Anlage ist es möglich das Verfahren effizient durchzuführen.

Als Verdampfer kann ein Fallfilmverdampfer vorgesehenwerden. Ein derartiger Verdampfer hat den Vorteil, daß die zugeführte Wärme optimal ausgenützt wird. Auch ein Verdampfen im Vakuum ist möglich.

Weiters kann als Verdampfer ein Dünnschichtverdampfer vorgesehenwerden. Auch mit einem derartigen Verdampfer ist ein Verdampfen im Vakuum möglich. Darüber hinaus werden mit einer solchen Einrichtung beste Ergebnisse erzielt.

Ferner kann als Verdampfer ein Rotationsfilmverdampfer vorgesehen werden. Durch die Zentrifugalkräfte des Rotationsfilmverdampfers ist der Verdampferfilm besonders dünnschichtig, so daß die Anlage mit höchster Effizienz und die Energie optimal ausnützend betrieben werden kann.

Es ist auch möglich, der Destilliereinrichtung eine Scheideeinrichtung nachzuschalten. Durch den Einsatz einer Scheideeinrichtung können die Rückstände, wie beispielsweise Glycerin einfach erfaßt und die Qualität bestimmt werden. Entsprechend der festgestellten Qualität wird die weitere Vorgangsweise festgelegt.

Gemäß einer Weiterbildung der Erfindung ist der Einrichtung zum Trennen der Phasen des Emulgates eine Scheideeinrichtung nachgeschaltet und die Scheideeinrichtung ist mit der Verbindungsleitung vom Behälter der Fette zur Reaktionsstrecke verbunden. Durch den Einsatz einer Scheideeinrichtung können die Rückstände, wie beispielsweise Glycerin einfach erfaßt und die Qualität bestimmt werden. Entsprechend der festgestellten Qualität wird die weitere Vorgangsweise festgelegt. Sollte bei der Analyse der in der Scheideeinrichtung befindlichen Stoffe festgestellt werden, daß noch nicht umgeesterte Fette vorhanden sind, werden diese einer weiteren Umesterung unterworfen. Die Ausbeute wird entsprechend erhöht.

Zum Einbringen der Flüssigkeit in die Reaktionsstrecke kann eine Pumpe, insbesondere eine Hochdruckpumpe vorgesehen werden. Die Integration einer Hochdruckpumpe hat sich vorteilhafterweise deshalb bewährt, da die Turbulenzen für die Umesterung eine hohe Dynamik und damit eine große Grenzfläche erzielen.

Nach einer Weiterbildung der Erfindung verdampft das überschüssige Methanol in einem Entspannungsreaktor. Durch diesen Schritt wird der Methylester methanolfrei gemacht.

Die Erfindung wird an Hand eines in der Zeichnung dargestellten Ausführungsbeispieles näher erläutert. Es zeigen:
Fig. 1 ein Fließschema des Verfahrensablaufes mit einer Destilliereinrichtung und
Fig. 2 ein Fließschema des Verfahrensablaufes mit einer Filtrationseinrichtung.

Einführend sei festgehalten, daß in der beschriebenen Ausführungsform gleiche Teile bzw. Zustände mit gleichen Bezugszeichen bzw. gleichen Bauteilbezeichnungen versehen werden, wobei die in der gesamten Beschreibung enthaltenen Offenbarungen sinngemäß auf gleiche Teile bzw. Zustände mit gleichen Bezugszeichen bzw. gleichen Bauteilbezeichnungen übertragen werden können. Weiters können auch Einzelmerkmale aus dem gezeigten Ausführungsbeispiel für sich eigenständige, erfindungsgemäße Lösungen darstellen.

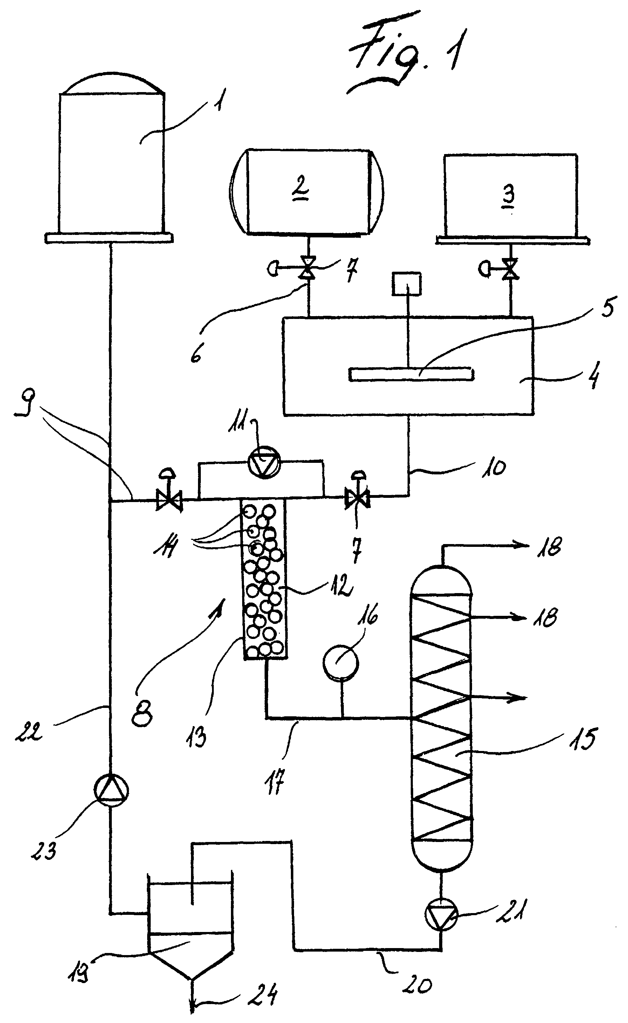

Gemäß der Fig. 1 beinhaltet der Behälter 1 höhere, gesättigte und ungesättigte Fette pflanzlichen und/oder tierischen Ursprungs. Ferner ist ein Vorratsbehälter 2 für eine starke Lauge, insbesondere für eine Kalilauge, und ein weiterer Vorratsbehälter 3 für den Alkohol, insbesondere für Methanol vorgesehen. Die Lauge wird in Alkohol gelöst, wobei dieser Vorgang im Mischbehälter 4 erfolgt. Zur Mischung der Lauge im Alkohol ist im Mischbehälter 4 ein Mischer 5 angeordnet. In den Zuleitungen 6 der Vorratsbehälter 2, 3 sind entsprechende Steuerventile 7 vorgesehen. Der Behälter 1 mit den Fetten und der Mischbehälter 4 sind über Verbindungsrohre 9 bzw. 10 mit der Umesterungsstrecke 8 verbunden. In den Verbindungsrohren 9 bzw. 10 sind weitere Steuerventile 7 angeordnet und zum Einbringen der Fette bzw. der Lösung in die Umesterungsstrecke 8 ist eine Hochdruckpumpe 11 vorgesehen.

Die Umesterungs- bzw. Reaktionsstrecke 8 besteht aus einem Statikmixer 12, der in diesem Fall aus einem Rohrl3 gebildet ist, das mit verschieden großen Kugeln 14 gefüllt ist. In dem Rohr 13 können auch weitere Einbauten, wie Leitbleche, Propeller. od. dgl. angeordnet sein. Der Statikmixer 12 verwirbelt die vom Dreifachester zum Einfachester umzuesternde Flüssigkeit durch hohe bzw. starke Turbulenzen. Dadurch werden die Phasengrenzflächen sehr stark vergrößert. Dies erfolgt dadurch, daß die Tropfengröße der umzuesternden Flüssigkeit durch die Turbulenzen stark verkleinert werden und so die Oberfläche stark vergrößert wird. Da die Umesterung eine Grenzflächenreaktion ist, wird durch die vergrößerte Oberfläche die Reaktionsgeschwindigkeit entsprechend erhöht, so daß sich das chemische Gleichgewicht sehr rasch einstellt.

Um nun die Reaktionszeit noch weiter zu beeinflussen, wird der Prozeßablauf bei bestimmten Temperaturen durchgeführt bzw. beginnt der Prozeßablauf mit hohem Druck, der im Zuge des Prozesses abgebaut wird.

Die Vergrößerung der Phasengrenzflächen könnte aber auch über Ultraschall erfolgen. Es ist daher durchaus denkbar, daß die Reaktionsstrecke mit einem Ultraschallgerät versehen wird.

Die sich im chemischen Gleichgewicht befindliche Flüssigkeit wird über eine Leitung 17 einer Destilliereinrichtung 15 zugeführt. Gegebenenfalls kann vorder Destilliereinrichtung 15 in der Leitung 17 eine Einrichtung 16 zur Entfernung von Methanol, beispielsweise ein Fallfilmverdampfer, vorgesehen werden.

Die Destilliereinrichtung 15 besteht mindestens aus einem Verdampfer und einem Kondensator, wobei die Destilliereinrichtung 15 als Vakuumdestillation ausgestaltet ist. Entsprechend den Stufen 18 der Destilliereinrichtung 15 werden die einzelnen Stoffe, wie eben der Fettsäuremethylester, abgezogen.

Natürlich können die verschiedensten Verdampfer Anwendung finden. So können beispielsweise Fallfilmverdampfer, Dünnschichtverdampfer, Rotationsfilmverdampfer od. dgl. Verwendung finden. Darüber hinaus umfaßt die Destilliereinrichtung 15 auch eine fraktionierte Kondensation. Durch die Ausbildung der Destilliereinrichtung 15 kann der Reinheitsgrad des Fettsäuremethylesters beeinflußt werden.
Die nicht umgeesterten Anteile werden separiert und in einer Scheideeinrichtung 19, die über eine Leitung 20 mit der Destilliereinrichtung 15 verbunden ist, gesammelt. Zum Einbringen dieser Anteile in die Scheideeinrichtung 19 ist in der Leitung 20 eine Pumpe 21 vorgesehen. In der Scheideeinrichtung 19 werden diese Anteile analysiert und entsprechend weiterverarbeitet. Gegebenenfalls wird ein Teil des nicht umgeesterten Anteiles über die Leitung 22, die eine Pumpe 23 aufweist, wieder den Fetten vor der Reaktionsstrecke 8 zugeführt. Bestimmte Anteile werden aus der Scheideeinrichtung 19 über eine Ausbringvorrichtung 24 abgezogen.

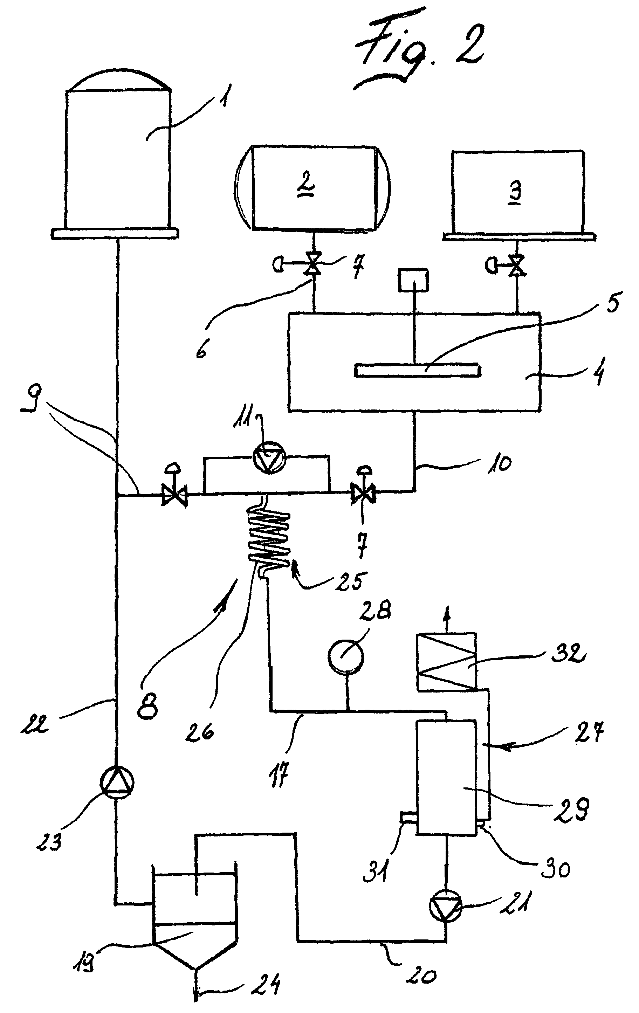

Gemäß der Fig.2 beinhaltet der Behälter 1 wieder höhere, gesättigte und ungesättigte Fette pflanzlichen und/oder tierischen Ursprungs. Ferner ist auch wieder ein Vorratsbehälter 2 für eine starke Lauge, insbesondere für eine Kalilauge, und ein weiterer Vorratsbehälter 3 für den Alkohol, insbesondere für Methanol vorgesehen. Die Lauge wird in Alkohol gelöst, wobei dieser Vorgang im Mischbehälter 4 erfolgt. Zur Mischung der Lauge im Alkohol ist im Mischbehälter 4 ein Mischer 5 angeordnet. In den Zuleitungen 6 der Vorratsbehälter 2, 3 sind entsprechende Steuerventile 7 vorgesehen. Der Behälter 1 mit den Fetten und der Mischbehälter 4 sind über Verbindungsrohre 9 bzw. 10 mit der Umesterungsstrecke 8 verbunden. In den Verbindungsrohren 9 bzw. 10 sind weitere Steuerventile 7 angeordnet und zum Einbringen der Fette bzw. der Lösung in die Umesterungsstrecke 8 ist eine Hochdruckpumpe 11 vorgesehen.

Die Umesterungs- bzw. Reaktionsstrecke 8 besteht aus einem dynamischen Emulgator 25, der in diesem Fall aus einem gewendeldeten Rohr 26 gebildet ist, das mit verschieden großen Kugeln gefüllt ist. In dem Rohr 26 können auch wieder weitere Einbauten, wie Leitbleche, Propeller, Widerstandskörper od. dgl. angeordnet sein. Der Emulgator 25 verwirbelt die vom Dreifachester zum Einfachester umzuesternde Flüssigkeit durch hohe bzw. starke Turbulenzen. Dadurch werden die Phasengrenzflächen sehr stark vergrößert. Dies erfolgt dadurch, daß die Tropfengröße der umzuesternden Flüssigkeit durch die Turbulenzen stark verkleinert werden und so die Oberfläche stark vergrößert wird. Da die Umesterung eine Grenzflächenreaktion ist, wird durch die vergrößerte Oberfläche die Reaktionsgeschwindigkeit entsprechend erhöht, so daß sich das chemische Gleichgewicht sehr rasch einstellt.

Statt des dynamischer Emulgator 25 könnte auch ein Spaltemulgator oder ein Turbulator bzw. eine Mischform von Spaltemulgator und Turbulator oder auch ein Kavitationsemulgator vorgesehen sein. Natürlich wäre es auch denkbar zwei oder mehrere Emulgatoren in Serie oder parallel vorzusehen. Die Vergrößerung der Phasengrenzflächen könnte aber auch über Ultraschall erfolgen. Es ist daher durchaus denkbar. daß die Reaktionsstrecke mit einem Ultraschallgerät versehen wird.

Um nun die Reaktionszeit noch weiter zu beeinflussen, wird der Prozeßablauf bei bestimmten Temperaturen, beispielsweise 40-70° C, durchgeführt bzw. beginnt der Prozeßablauf mit hohem Druck, der vorzugsweise im Zuge des Prozesses abgebaut wird. Es könnte aber auch ein Temperaturbereich gewählt werden, in dem das überschüssige Methanol in einem Entspannungsreaktor verdampft und durch diesen Schritt der Methylester methanolfrei gemacht wird.

Die sich im chemischen Gleichgewicht befindliche Flüssigkeit wird über eine Leitung 17 einer Einrichtung 27 zum Trennen der Phasen der Mischung zugeführt. Gegebenenfalls kann vor der Einrichtung 27 in der Leitung 17 eine Einrichtung 28 zur Anzeige des Druckes vorgesehen werden.

Die Einrichtung 27 besteht mindestens aus einer Filtrationseinrichtung 29. Entsprechend der Ausbildung der Einrichtung 27 werden die einzelnen Phasen, wie eben der Fettsäuremethylester. abgetrennt und an der Ausbringstelle 30 abgezogen. Die Glycerin-Phase kann an der Ausbringstelle 31 zu ihrer weiteren Verwendung entnommen werden.

Durch die Ausbildung der Filtrationseinrichtung 29 kann der Reinheitsgrad des Fettsäuremethylesters beeinflußt werden. Natürlich könnte auch der Fettsäuremethylester in einer nachgeschalteten Einrichtung zur Reinigung 32 weiterbehandelt werden. Ebenso ist es denkbar, daß die Filtrationseinrichtung 29 ein Molekularsiebfilter umfaßt.

Die Filtrationseinrichtung 29 ist ein Oberflächenfilter, das in Membrantechnologie hergestellt ist und aus einem porösen Trägerkörper, beispielsweise aus Aluminiumoxid, der als Rohr ausgebildet ist und einer auf den Trägerkörper aufgebrachten Schicht, beispielsweise eine Titandioxidschicht, besteht. Die Schicht kann amphotere Eigenschaften aufweisen, wobei die Porengröße im Nanobereich liegt.

Die nicht umgeesterten Anteile werden separiert und in einer Scheideeinrichtung 19, die über eine Leitung 20 mit der Destilliereinrichtung 15 verbunden ist, gesammelt. Zum Einbringen dieser Anteile in die Scheideeinrichtung 19 ist in der Leitung 20 eine Pumpe 30 21 vorgesehen. In der Scheideeinrichtung 19 werden diese Anteile analysiert und entsprechend weiterverarbeitet. Gegebenenfalls wird ein Teil des nicht umgeesterten Anteiles über die Leitung 22, die eine Pumpe 23 aufweist, wieder den Fetten vor der Reaktionsstrecke 8 zugeführt. Bestimmte Anteile werden aus der Scheideeinrichtung 19 über eine Ausbringvorrichtung 24 abgezogen.

Abschließend sei der Ordnung halber darauf hingewiesen, daß in der Zeichnung einzelne Bauteile und Baugruppen zum besseren Verständnis der Erfindung unpropotional und maßstäblich verzerrt dargestellt sind.

## Patentansprüche

1. Verfahren zur Herstellung von Fettsäuremethylester, insbesondere Dieselkraftstoff für Fahrzeuge, wobei höhere, gesättigte und ungesättigte Fette pflanzlichen und/oder tierischen Ursprungs mit einer Lösung von starken Laugen, insbesondere Kalilauge, in Alkohol, insbesondere in Methanol, versetzt werden und die beiden Komponenten in einer Reaktionsstrecke durch Emulgierung der Mischung über eine Grenzflächenreaktion, wobei die Fette in Fettsäuremethylester umgeestert werden, ihren chemischen Gleichgewichtszustand erreichen und nach Erreichen des Gleichgewichtszustandes die Rückstände, wie Triglyceride, Glycerin, Seife, nicht umgeesterte Fette u. dgl., vom Fettsäuremethylester getrennt werden, **dadurch gekennzeichnet, daß** in der Reaktionsstrecke (8) die Phasengrenzflächen der Mischung durch hohe bzw. starke, dynamische Turbulenzen vergrößert werden und daß die Umesterung unter hohem Druck durchgeführt wird, wobei gegebenenfalls die Umesterung unter hohem Druck beginnt und der Druck während der Umesterung abgebaut wird.

2. Verfahren nach Anspruch 1, **dadurch gekennzeichnet, daß** die hohen bzw. starken dynamischen Turbulenzen durch physikalische Kräfte, beispielsweise mechanische Scherkräfte, wie einen Spaltemulsionsvorgang oder durch dynamische Emulgierung, beispielsweise über einen Turbulator, oder durch Kavitationsemulgation oder durch Ultraschall, erzeugt werden.

3. Verfahren nach Anspruch 1 oder 2, **dadurch gekennzeichnet, daß** die Trennung der Phasen des Emulgates auf physikalischem Weg und nach der Trennung der Phasen des Emulgates eine Reinigung (32) des Fettsäuremethylesters durchgeführt wird, wobei die Trennung der Phasen des Emulgates unter Ausnützung der Oberflächenkräfte, insbesondere über eine, gegebenenfalls mehrstufige, Filtration erfolgt und zur Filtrierung, gegebenenfalls lipophile und/oder hydrophile und/oder amphotere, Filter aus der Membrantechnologie, beispielsweise aus dem Ultraund/oder Nano- und/oder Mikrobereich, eingesetzt werden.

4. Verfahren nach mindestens einem der Ansprüche 1 bis 3, **dadurch gekennzeichnet, daß** die Trennung der Phasen des Emulgates auf chemischem Weg durchgeführt wird, wobei nach Einstellung des Gleichgewichtes, gegebenenfalls nach der Trennung der Phasen des Emulgates, eine mehrstufige Destillation durchgeführt wird, die mindestens eine Vakuumdestillation oder mindestens eine Verdampfung, insbesondere eine Fallfilmverdampfung oder eine Dünnschichtverdampfung oder eine fraktionierte Kondensation umfaßt.

5. Verfahren nach mindestens einem der Ansprüche 1 bis 4, **dadurch gekennzeichnet, daß** die nicht umgeesterten Anteile separiert und vor der Reaktionsstrecke (8) den Fetten wieder zugeführt werden.

6. Anlage zur Durchführung des Verfahrens nach mindestens einem der Ansprüche 1 bis 5, **dadurch gekennzeichnet, daß** mindestens ein Behälter (1) für die Fette sowie je ein Vorratsbehälter (2,3) für die starke Lauge und für den Alkohol bzw. mindestens ein Mischbehälter (4) für deren Lösung vorgesehen sind und mindestens der Behälter (1) und der Mischbehälter (4) mit der Reaktionsstrecke (8) verbunden sind und daß der Reaktionsstrecke (8) eine Einrichtung (27) zur Trennung der Phasen des Emulgates nachgeschaltet ist.

7. Anlage nach Anspruch 6, **dadurch gekennzeichnet, daß** die Reaktionsstrecke aus einem Statikmixer besteht, wobei der Statikmixer, vorzugsweise ein mit verschieden großen Kugeln gefülltes Rohr (26) und/oder gegebenenfalls Einbauten wie Leitbleche, Propeller, Widerstandskörper, od. dgl. aufweist.

8. Anlage nach Anspruch 6 oder 7, **dadurch gekennzeichnet, daß** in der Reaktionsstrecke (8) ein dynamischer Emulgator (25), beispielsweise ein Kavitationsemulgator oder ein Spaltemulgator oder ein Turbulator oder eine Mischform eines Spaltemulgators und eines Turbulators vorgesehen ist, die beispielsweise aus zwei sich relativ zueinander bewegender Scheiben besteht und die Zuführung des Emulgates mittig einer Scheibe erfolgt.

9. Anlage nach mindestens einem der Ansprüche 6 bis 8, **dadurch gekennzeichnet, daß** in der Reaktionsstrecke (8) ein Ultraschallgerät, vorgesehen ist.

10. Anlage nach mindestens einem der Ansprüche 6 bis 9, **dadurch gekennzeichnet, daß** die Einrichtung (27) zum Trennen der Phasen des Emulgates eine, gegebenenfalls mehrstufige, Filtrationseinrichtung (29) ist, wobei als Filtrationseinrichtung (29) ein Oberflächenfilter in Membrantechnologie vorgesehen ist und der Oberflächenfilter aus einem porösen Trägerkörper und einer auf diesen Trägerkörper aufgebrachten Schicht, die als Membran wirkt, besteht und der Trägerkörper als Rohr ausgebildet ist und beispielsweise aus Aluminiumoxid, porösem Glas oder Silikaten besteht und die als Membran wirkende Schicht lipophile und/oder hydrophile und /oder amphotere Eigenschaften aufweist, wobei die als Membran wirkende Schicht eine Keramikmembran ist, die beispielsweise aus Titandioxid, Zirkondioxid, o. dgl. besteht und Poren im Nano- und/oder Mikrobereich, insbesondere mit einer Größe von 5-200 nm, aufweist oder die Filtrationseinrichtung (29) ein Molekularsiebfilter bzw. eine Molekularsiebmembran ist bzw. umfaßt.

11. Anlage nach mindestens einem der Ansprüche 6 bis 10, **dadurch gekennzeichnet, daß** der Einrichtung (27) zum Trennen der Phasen des Emulgates eine Scheideeinrichtung (19) nachgeschaltet ist und die Scheideeinrichtung (19) mit der Verbindungsleitung (9) vom Behälter (1) der Fette zur Reaktionsstrecke (8) verbunden ist.

12. Anlage nach mindestens einem der Ansprüche 6 bis 11, **dadurch gekennzeichnet, daß** das überschüssige Methanol in einem Entspannungsreaktor verdampft.

## Claims

1. Method for producing fatty acid methyl ester, more particularly diesel fuel for vehicles, whereby saturated and unsaturated higher fatty substances of vegetable and/or animal origin are compounded with a potent alkaline solution, particularly a potassium solution, and dissolved in alcohol, particularly methanol, and the two components reach a chemical balance state through emulsification of the mixture in a reaction section with a phase separation section, whereby the fats are transesterified into fatty acid methyl ester, and on reaching the chemical balance state the residues such as triglycerides, glycerine, soap, non-transesterified fats, etc. are separated from the fatty acid methyl ester, **characterised by** the fact that the border surfaces of the mixture are enlarged by high or powerful dynamic turbulence in the reaction section (8), whereby the transesterification process may begin under high pressure and the pressure be reduced during the transesterification.

2. Method according to Claim 1, **characterised by** the fact that the high or powerful dynamic turbulence is produced by physical forces, for example mechanical shear forces, such as crack emulsification, or by dynamic emulsification, for example using a turbulator, or by cavitation emulsification, or by ultrasound.

3. Method according to Claim 1 or 2, **characterised by** the fact that separation of the phases of the emulsion is carried out physically, and that purification (32) of the fatty acid methyl ester is carried out after separation of the phases, whereby the separation of the phases of the emulsion is carried out using the surface forces, in particular by means of filtration, possibly multiphase filtration, and that lipophilic and/or hydrophilic and/or amphoteric filters using membrane technology, for example in the ultra and/or nano and/or micro range, are used for filtration.

4. Method according to at least one of Claims 1 to 3, **characterised by** the fact that the phases of the emulsion are separated by chemical means, whereby after reaching chemical balance state, possibly after separation of the phases of the emulsion, multiphase distillation is performed, which comprises at least one vacuum distillation or at least one evaporation, in particular down-flow evaporation or thin-layer evaporation, or fractionated condensation.

5. Method according to at least one of Claims 1 to 4, **characterised by** the fact that non-transesterified fats are separated and returned to the fats upstream from the reaction section (8).

6. Equipment for realisation of the method according to at least one of Claims 1 to 5, **characterised by** the fact that there is at least one container (1) for the fats, and one tank each (2, 3) for the potent alkaline solution and the alcohol, as well as at least one mixing vessel (4) for compounding them, and that at least the container (1) and the mixing vessel (4) are connected to the reaction section (8), and that there is a unit (27) for separation of the phases of the emulsion downstream from the reaction section (8).

7. Equipment according to Claim 6, **characterised by** the fact that the reaction section consists of a static mixer, whereby the static mixer is fitted preferably with a pipe (26) filled with balls of various sizes and/or devices such as baffles, propellers, resistors, etc.

8. Equipment according to Claim 6 or 7, **characterised by** the fact that there is a dynamic emulsifier (25) in the reaction section (8), for example a cavitation emulsifier or a crack emulsifier or a turbulator, or a mixed form of crack emulsifier and turbulator, for example consisting of two discs moving in relation to one another, whereby the emulsion is introduced in the middle of one of the discs.

9. Equipment according to at least one of Claims 6 to 8, **characterised by** the fact that there is an ultrasound device in the reaction section (8).

10. Equipment according to at least one of Claims 6 to 9, **characterised by** the fact that the unit (27) for separating the phases of the emulsion is a filtration unit (29), possibly a multiphase filtration unit, whereby a surface filter using membrane technology is used as filtration unit (29), and the surface filter consists of a porous carrier and a layer applied to this carrier which acts as membrane, and the carrier is designed as a pipe and made, for example, of aluminium oxide, porous glass or silicates, and the layer acting as membrane has lipophilic and/or hydrophilic and/or amphoteric properties, whereby the layer acting as membrane is a ceramic membrane made, for example, of titanium dioxide, zirconium dioxide, etc., and has pore sizes in the nano and/or micro range, in particular with a pore size of 5-200 nm, or the filtration unit (29) is or comprises a molecular sieve filter or molecular sieve membrane.

11. Equipment according to at least one of Claims 6 to 10, **characterised by** the fact that there is a separation unit (19) downstream from the unit (27) for separating the phases of the emulsion, and that the separation unit (19) is connected to the reaction section (8) by a connecting pipe (9) from the container (1) for the fats.

12. Equipment according to at least one of Claims 6 to 11, **characterised by** the fact that the surplus methanol is evaporated in a flash reactor.

## Revendications

1. Méthode de production d'ester méthylique d'acide gras, en particulier de carburant diesel pour véhicules ; à cette occasion des matières grasses supérieures, saturées et non saturées d'origine végétale et/ou animale sont mélangées avec une forte solution de lessive alcaline, en particulier de la potasse caustique, dans de l'alcool, en particulier de l'alcool méthylique ; les deux composants atteignent leur équilibre chimique par voie de réaction, à savoir un émulsionnement du mélange par une réaction interfaciale durant laquelle les matières grasses sont transestérifiées en ester méthylique d'acide gras ; après avoir atteint leur équilibre chimique les résidus comme les triglycérides, la glycérine, le savon, les matières grasses non transestérifiées etc. sont séparés de l'ester méthylique d'acide gras ; la méthode de production est **caractérisée par le fait que** pendant la voie de réaction (8) les interphases du mélange sont agrandies par des turbulences élevées, fortes et dynamiques et que la transestérification est effectuée sous pression élevée ; le cas échéant, la transestérification débute avec une pression élevée qui est réduite tout au long de la transestérification.

2. Méthode conformément à la revendication 1, **caractérisée par le fait que** les turbulences élevées, fortes, voire dynamiques sont produites par les forces physiques, par exemple des efforts de cisaillement mécanique comme ceux d'une réaction d'émulsion par clivage ou par un agent émulsionnant dynamique, par exemple par un turbulateur, par un agent émulsionnant par cavitation ou par ultra-son.

3. Méthode conformément à la revendication 1 ou 2, **caractérisée par le fait que** la séparation des phases de l'émulsion est effectuée de manière physique et qu'après la séparation des phases de l'émulsion un nettoyage (32) de l'ester méthylique d'acide gras est effectué ; la séparation des phases de l'émulsion est effectuée en utilisant les forces de surface, notamment au moyen d'une filtration, si nécessaire à plusieurs étapes ; lors de la filtration sont utilisés, le cas échéant, des filtres lipophiles et/ou hydrophiles et/ou amphotères tels qu'ils sont connus dans la technologie de membranes, par exemple dans les domaines ultra, nano et micro.

4. Méthode selon au moins une des revendications 1 à 3, **caractérisée par le fait que** la séparation des phases de l'émulsionnant est effectuée par voie chimique ; pour cela, une distillation polyétagée est effectuée après avoir atteint l'équilibre, le cas échéant, après la séparation des phases de l'émulsionnant, comprenant au moins une distillation dans le vide ou au moins une vaporisation, notamment une évaporation à courant descendant ou une évaporation à couche mince ou une condensation fractionnée.

5. Méthode conformément à au moins une des revendications 1 à 4, **caractérisée par** la séparation des composants non transestérifiés et par leur introduction aux matières grasses avant la voie de réaction (8).

6. Installation pour réaliser la méthode selon au moins une des revendications de 1 à 5, **caractérisée par le fait qu'**elle comporte au moins un réservoir (1) pour les matières grasses ainsi qu'un réservoir (2, 3) pour la forte lessive alcaline et l'alcool et au moins un réservoir (4) pour leur dissolution ; au moins le réservoir (1) et le réservoir de dissolution (4) sont connectés à la voie de réaction (8) et la voie de réaction (8) est suivie d'un dispositif (27) de séparation des phases de l'émulsionnant.

7. Installation selon la revendication 6, **caractérisée par le fait que** la voie de réaction est composée d'un mixeur statique ; de préférence celui-ci comporte un tuyau (26) rempli de boules de différentes tailles et des objets installés comme des tôles de guidage, des hélices, des corps de résistance ou semblables.

8. Installation selon la revendication 6 ou 7, **caractérisée par le fait que** dans la voie de réaction (8) un agent émulsionnant dynamique est prévu (25), par exemple un agent émulsionnant de cavitation ou un agent émulsionnant de craquage ou un turbulateur ou une forme mixte d'un agent émulsionnant de craquage et d'un turbulateur, qui consiste en deux disques ayant un mouvement relatif l'un par rapport à l'autre et que l'alimentation de l'agent émulsionnant se fait au milieu d'un des disques.

9. Installation selon l'une des revendications 6 à 8, **caractérisée par le fait qu'**un appareil à ultra-son est prévu dans la voie de réaction (8).

10. Installation selon au moins une des revendications 6 à 9, **caractérisée par** une installation de filtration (29), le cas échéant polyétagée, servant comme installation (27) permettant la séparation des phases de l'agent émulsionnant ; pour l'installation de filtration (29) est prévu un filtre de surface en technologie à membrane, le filtre de surface est constitué d'un corps porteur poreux et d'une couche appliquée sur ce filtre poreux qui agit comme membrane ; ce corps porteur constitué en forme de tuyau est fabriqué par exemple en oxyde d'aluminium, en verre poreux ou en silicates et la couche agissant comme membrane est dotée de caractéristiques lipophiles et/ou hydrophiles et/ou amphotères ; cette couche agissant comme membrane est une membrane en céramique composée par exemple en blanc de titane, en zircone ou une matière semblable ; elle possède des pores dans le domaine nano et/ou micro, en particulier d'une taille de 5 à 200 nm, et l'installation de filtration (29) est un filtre-tamis moléculaire ou une membrane-tamis moléculaire ou bien elle comprend un de ces composants.

11. Installation selon au moins une des revendications 6 à 10, **caractérisée par le fait que** l'installation (27) de séparation des phases de l'agent émulsionnant est suivie d'une installation de cloisonnement (19) ; cette installation de cloisonnement (19) est reliée à la conduite de raccordement (9) entre le réservoir (1) des matières grasses et la voie de réaction (8).

12. Installation selon l'une des revendications de 6 à 11, **caractérisée par le fait que** l'alcool méthylique redondant s'évapore dans un réacteur de détente.
